# EUROPEAN PATENT APPLICATION

(11) **EP 1 731 151 A1**
(43) Date of publication of application: **13.12.2006**
(21) Application number: 05720314.3
(22) Date of filing: 09.03.2005
(51) Int. Cl.: A61K 31/435, A61K 9/32, A61K 47/04, A61P 1/10

(54) **SOLID PHARMACEUTICAL PREPARATION CONTAINING SPARINGLY WATER-SOLUBLE DRUG**

(30) Priority: 10.03.2004 JP 2004067877
(71) Applicant: TAISHO PHARMACEUTICAL CO., LTD, Tokyo 170-8633 (JP)
(72) Inventor: WADA, Kaoru, Taisho Pharmaceutical Co., Ltd., Tokyo 1708633 (JP); ANDO, Shinji, Taisho Pharmaceutical Co., Ltd., Tokyo 1708633 (JP)
(74) Representative: HOFFMANN EITLE
(86) International application number: PCT/JP2005/004042
(87) International publication number: WO 2005/087230

(57) **Abstract**

Even when being released in a region from a lower small intestine to a large intestine with various coatings, bisacodyl is inadequately effective because of its hardness to dissolve due to its poor solubility, and thus a more drug should be contained. In the related art, there have been no techniques for allowing bisacodyl and dioctyl sodium sulfosuccinate to be simultaneously released in a region from a lower small intestine to a large intestine.

A solid formulation having a core particle containing bisacodyl and dioctyl sodium sulfosuccinate coated with an enteric coating which allows the drugs to be released in a region from a lower small intestine to a large intestine can be used as an excellent pharmaceutical product.

## Description

### TECHNICAL FIELD

The present invention relates to a poorly water-soluble drug-containing solid formulation, more specifically to a bisacodyl-containing solid formulation.

### BACKGROUND ART

Bisacodyl directly acts on a large intestinal mucosa and is used as a cathartic for stimulating defecation by enhancing intestinal peristaltic movement mainly in a form of a formulation for oral administration. It is known to be extremely insoluble in water.

There has been a problem that when being absorbed in a site before an upper small intestine, bisacodyl repeats its action due to enterohepatic circulation. It is, therefore, preferable to allow bisacodyl to act in a region from a lower small intestine to a large intestine.

Dioctyl sodium sulfosuccinate is known as a cathartic drug. It directly acts on a hard stool in a bowel and causes water permeation thereinto, to soften and swell it and then to promote excretion.

There has been conventionally described a technique for improving stability of a formulation combined with both bisacodyl and dioctyl sodium sulfosuccinate together (Patent Reference 1).

There have been reported in some documents that bisacodyl is coated with various coatings for releasing bisacodyl in a region from a lower small intestine to a large intestine (Patent References 2 to 4).

There have been, however, no techniques for allowing bisacodyl and dioctyl sodium sulfosuccinate to be simultaneously released in a region from a lower small intestine to a large intestine.

Patent Reference 1: Japanese Laid-open Patent Publication No. S57-99,521.
Patent Reference 2: Japanese Laid-open Patent Publication No. H10-203,983.
Patent Reference 3: published Japanese translation of PCT international publication for patent application No. H11-506,432.
Patent Reference 4: published Japanese translation of PCT international publication for patent application No. H11-506,433.

### DISCLOSURE OF THE INVENTION

### PROBLEM TO BE RESOLVED BY THE INVENTION

Even when being released in a region from a lower small intestine to a large intestine by applying various coatings, bisacodyl is inadequately effective due to its poor solubility, resulting in unsatisfactory cathartic effect.

An objective of this invention is to provide a formulation containing bisacodyl which is adequately effective.

### MEANS OF SOLVING PROBLEM

After intense investigation for solving the above problems, the present inventors have found that a core combined with both bisacodyl and dioctyl sodium sulfosuccinate together can be coated with an enteric coating which allows the core to be released in a region from a lower small intestine to a large intestine, to improve a solubility of bisacodyl in its action site and to synergistically provide higher drug efficacy at the same drug dosages, and have finally achieved the present invention.

This invention provides:
[1] a solid formulation comprising a core containing bisacodyl and dioctyl sodium sulfosuccinate coated with an enteric coating which allows the drugs to be released in a region from a lower small intestine to a large intestine;
[2] the solid formulation as described in [1] wherein the core contains light anhydrous silicic acid; and
[3] the solid formulation as described in [1] or [2] wherein the formulation is insoluble in a first fluid for 2 hours and has a disintegration time of more than 60 minutes in a second fluid in accordance with Disintegration Test, Japanese Pharmacopoeia.
   In the present invention, a core refers to an object to be coated with an enteric coating including granules prepared by granulating a powder containing dioctyl sodium sulfosuccinate and bisacodyl, a plain tablet, and also a powder and a gel.

An enteric coating herein includes films for granules or a plain tablet and also a capsule which is dissolved in an intestine. When using a capsule, an enteric capsule material is filled with granules as a core to give a capsule.

In the present invention, an enteric coating which releases a drug in a region from a lower small intestine to a large intestine refers to a coating which is insoluble in a first fluid for 2 hours and has a disintegration time of more than 60 minutes in a second fluid in accordance with Disintegration Test, Japanese Pharmacopoeia. An enteric film used in the inventionmaybe anymaterial capable of releasing a drug in a region from a lower small intestine to a large intestine. For example, the objective of the present invention can be achieved using a multilayer coating including an enteric polymer such as cellulose acetate phthalate as described in Patent References 2 to 4.

A content of bisacodyl in this invention is preferably 0.75 to 45 mg, more preferably 5 to 15 mg per dose in the light of cathartic effect. When its dosage form is a tablet, a content is preferably 0.75 to 15 mg, more preferably 3 to 8 mg per tablet.

In the present invention, a content of dioctyl sodium sulfosuccinate is preferably 3 to 360 mg, more preferably 8 to 30 mg per a dose of a bisacodyl-containing formulation in the light of synergy with bisacodyl. When its dosage form is a tablet, a content is preferably 3 to 120 mg, more preferably 5 to 30 mg per tablet.

In the present invention, a coating amount when a core is directly coated with an enteric coating is preferably 1 to 30 % by mass to a core mass.

A core used in the present invention is prepared as follows.

Dioctyl sodium sulfosuccinate is dissolved in a solvent such as water and ethanol to prepare a solution. By spraying or in one portion, the solution is added to andmixedwith an excipient such as light anhydrous silicic acid and a powder thus obtained is granulated into a granular powder.

To the granular powder obtained are added bisacodyl and, if necessary, another pharmaceutical ingredient or an additive which is commonly used for preparing a pharmaceutical product, and the mixture can be subsequently mixed, pulverized or granulated as usual to provide a core in a form of granules, tablets or the like.

The core thus obtained can be coated with an enteric coating which allows a drug to be released in a region from a lower intestine to a large intestine by a known process, to provide a solid formulation of the present invention.

For preparing a core in this invention, a powder preferably contains a water-soluble polymer commonly used as a binder.

Examples of a water-soluble polymer include hydroxypropylcellulose, polyvinylpyrrolidone and hydroxypropylmethylcellulose. A content of the water-soluble polymer is preferably about 10 wt % in the total amount of the core particle.

For preparing a solid formulation of this invention, a core preferably contains light anhydrous silicic acid in the light of its properties. A content of light anhydrous silicic acid is preferably 0.5 to 1.5 parts by mass, more preferably 0.6 to 1.3 parts by mass to one part by mass of dioctyl sodium sulfosuccinate.

A lower small intestine where is a drug-release site for the formulation of the present invention corresponds to an entrance to a large intestine and thus a gastrointestinal tract where a small amount of digestive fluid is secreted. In terms of an amount of digestive fluid in a human gastrointestinal tract, it generally reduces from a small intestine toward a large intestine. Therefore, when a drug is released in a region from a lower small intestine to a large intestine, a solubility of a drug itself often has an effect on its efficacy. Dioctyl sodium sulfosuccinate contained in the formulation of this invention also acts as a surfactant and when being combined with a drug, can thus improve solubility of the drug. In particular, it is very effective for dissolving a poorly-soluble drug such as bisacodyl in a site secreting a small amount of digestive fluid.

In this invention, bisacodyl and dioctyl sodium sulfosuccinate are simultaneously released in a region from a lower small intestine to a large intestine to exhibit synergistic effect. For a common coating which is not a coating capable of allowing a drug to be released in a region from a lower small intestine to a large intestine, synergistic effect cannot be expected even when both ingredients are simultaneously administered orally because each ingredient is not available at a target site in an adequate level.

### EFFECT OF THE INVENTION

It has been found that a solid formulationof the present invention improves solubility of bisacodyl at its action site, resulting in synergistic improvement in cathartic effect.

### BRIEF DESCRIPTION OF THE DRAWINGS

FIG. 1 shows the results of a flow-through cell dissolution test for DSS and bisacodyl.
FIG. 2 shows the results of a flow-through cell dissolution test for bisacodyl.
FIG. 3 shows cathartic effect when DSS and bisacodyl are simultaneously administered, where an ordinate and an abscissa indicate catharsis incidence rates and samples, respectively.

### BEST MODE FOR CARRYING OUT THE INVENTION

This invention will be more specifically described with reference to Examples, Comparable Examples and Test Examples.

### Example 1

This formulation was prepared according to the composition shown in Table 1. It was a combination prepared by mixing bisacodyl as an active ingredient with lactose, hydroxypropylcellulose, lower-substituted hydroxypropylcellulose and light anhydrous silicic acid. Separately, another active ingredient, dioctyl sodium sulfosuccinate was dissolved in a mixture of ethanol and purified water, and the resulting granulation solution was sprayed onto the combination to prepare granules. After drying, the resulting granules were combined with light anhydrous silicic acid as powder, magnesium stearate and granulating lactose to prepare mixed granules, which was then tableted to prepare tablets with a mass of 50 mg/tablet. They were used as a core particle.

Next, a methacrylic acid copolymer (S, L mixture), magnesium stearate and castor oil were dispersed and dissolved in ethanol and acetone to prepare a coating solution, with which the core particles were coated in 8 mg/tablet to prepare a solid formulation of this invention.

### Comparative Example 1

Core particles were prepared as described in Example 1 without a coating, and was used as Comparative Example 1.

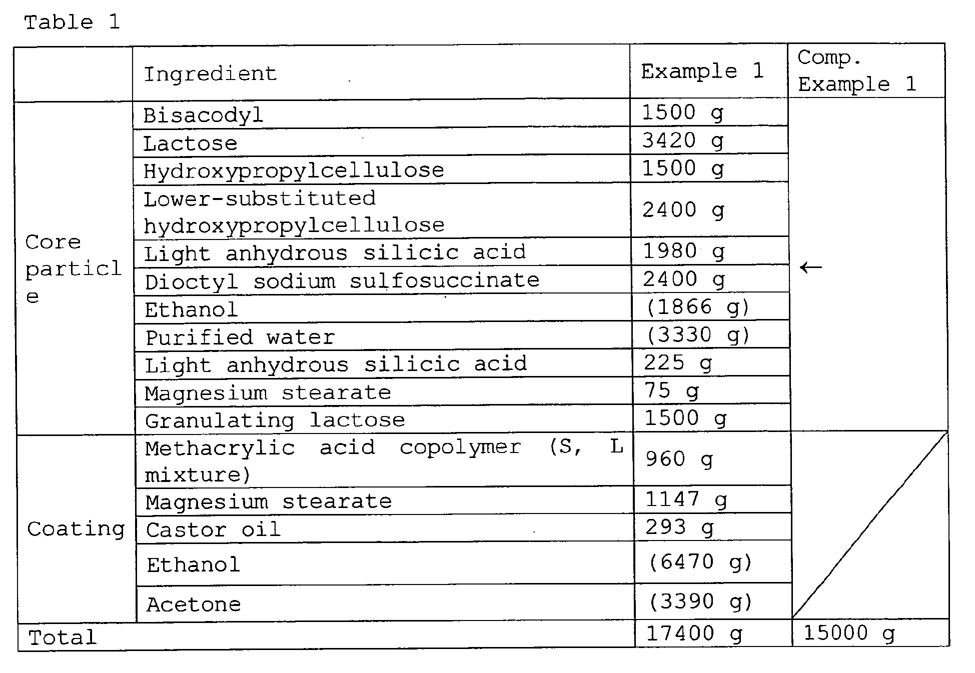

### Test Example 1

The formulations in Example 1 and Comparative Example 1 were evaluated by a dissolution test. The test method was in accordance with Method 3 (Flow-through cell method), Japanese Pharmacopoeia and the amount of eluted dioctyl sodium sulfosuccinate was determined by HPLC. This test is reproduction of behavior of a formulation in a human gastrointestinal tract because in this test acidity or alkalinity of a test solution fed into a cell is varied over time, and thus suitable for evaluating eluting properties of a site-targeting formulation. Table 2 shows acidity or alkalinity and an eluting time of a test solution properties.

**Table 2**

| Eluting time [min] | pH | Site in a human gastrointestinal tract |
|---|---|---|
| 0 to 30 | 1.2 | Stomach |
| 30 to 90 | 6.55 | Upper small intestine |
| 90 to 210 | 7.21 | Lower small intestine |
| 210 to 360 | 6.71 | Large intestine |

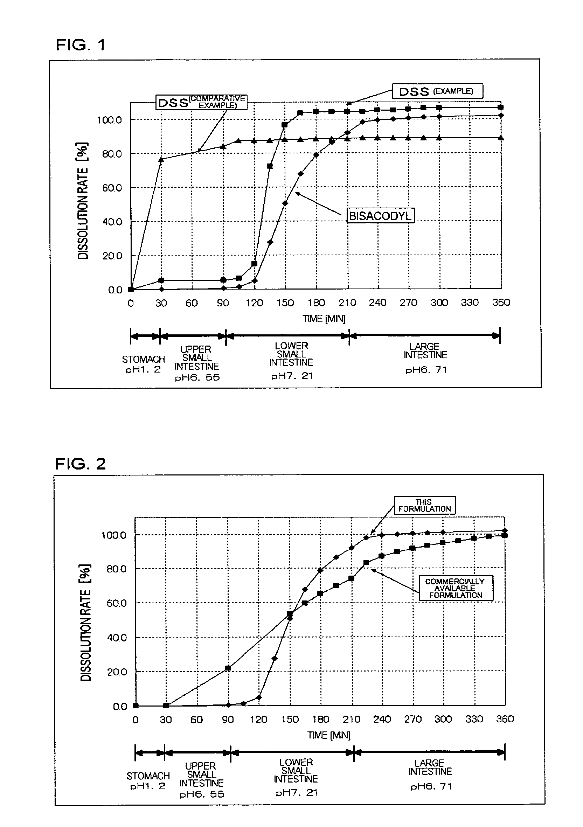

FIG. 1 shows a dissolution curve for dioctyl sodium sulfosuccinate.

As seen from the figure, the formulation in Example 1 initiated drug release in a high pH region simulating a lower small intestine, while the formulation in Comparative Example 1 containing core particles alone initiated drug release at pH 1.2 simulating a stomach.

Furthermore, similar evaluation for dissolution of bisacodyl from the formulation of Example 1 indicated that release of the drug was initiated in a lower small intestine as in dioctyl sodium sulfosuccinate.

### Test Example 2

This formulation is characterized in that drug release is initiated in a lower gastrointestinal tract in contrast to common enteric formulations, and is thus more effective as a cathartic. For demonstrating it, disintegration and dissolution properties were compared between this formulation and a common (commercially available) enteric formulation containing bisacodyl.

A disintegration time for the formulation in Example 1 was determined by Disintegration Method and Shaking Method in Japanese Pharmacopoeia. In the shaking method, about 10 mL of a test solution and one tablet were placed in a 50 mL volumetric flask. While the mixture was shaken by a shaker at about 100 strokes/min and an amplitude of 3 cm, a time taken for disintegration of the tablet was recorded. In this test, the first fluid, J. P. (pH 1.2), the second fluid J. P. (pH 6.8) and a test solution at pH 8.3 were used and for each solution, a disintegration time was determined. As a comparative example, a commercially available formulation was evaluated by the shaking method.

Table 3 shows a disintegration time for each test solution.

**Table 3**

| Test solution | Example 1 | | Commercially available formulation |
|---|---|---|---|
| | Disintegration method, J. P. | Shaking method | Shaking method |
| First fluid; pH 1.2 | Not disintegrated in a 120 min test period | Not disintegrated in a 120 min test period | Not disintegrated in a 120 min test period |
| Second fluid; pH 6.8 | Not disintegrated in 60 min | Not disintegrated in a 120 min test period | 31 min |
| pH 8.3 | 43 min | 70 min | 41 min |

As seen in this table, the test results in the shaking method indicate that this formulation is not disintegrated in the test solution at pH 6.8 in 120 min in contrast to the common enteric formulation. There is furthermore demonstrated that this formulation is not disintegrated in the second fluid at pH 6.8 in Disintegration Method, Japanese Pharmacopoeia, but disintegrated only in the test solution at pH 8.3. "Disintegration" as usedhereinmeans that an enteric coating is dissolved and thus release of an internal drug is initiated.

### Test Example 3

Drug release properties of Example 1 and a commercially available formulation were evaluated by a flow-through cell method using bisacodyl as a reference. The results are shown in FIG. 2.

As seen in this figure, this formulation did not initiate dissolution of bisacodyl in a pH range corresponding to a stomach and an upper small intestine, but initiated dissolution in a high pH range corresponding to a lower small intestine.

The above results of the disintegration and the dissolution tests demonstrate that this formulation disintegrates and initiate drug release in a higher pH range, that is, a lower small intestine, than a common enteric formulation.

### Test Example 4

Variation in solubility of bisacodyl was studied in the co-existence of bisacodyl and dioctyl sodium sulfosuccinate. Bisacodyl was dispersed in purified water and the dispersion was shaken under given conditions for 5 hours. The resulting liquid was centrifuged at 3000 min⁻¹ for 5 min. Then, the supernatant was collected and the amount of dissolved bisacodyl was determined by HPLC.

In a similar manner, bisacodyl was dispersed in solutions containing dioctyl sodium sulfosuccinate at 0.07%, 0.14% and 0.35 %. Each dispersion was shaken and centrifuged at 3000 min⁻¹ for 5 min. Then, the supernatant was collected and the amount of dissolved bisacodyl was determined by HPLC. The results are shown in Table 4.

**Table 4**

| Sample | | Concentration of DSS [%] | Solubility ofbisacodyl [mg/mL] | Proportion to control [%] |
|---|---|---|---|---|
| Control | Bisacodyl + purified water | 0 % | 0.00189 | - |
| Sample 1 | Bisacodyl + 0.07% DSS aq. | 0.07 % | 0.00362 | 191.5 % |
| Sample 2 | Bisacodyl + 0.14%DSS aq. | 0.14 % | 0.00451 | 238.6 % |
| Sample 3 | Bisacodyl + 0.35% DSS aq. | 0.35 % | 0.0127 | 672.0 % |

As seen from Table 4, it was found that in the co-existence of bisacodyl and dioctyl sodium sulfosuccinate, a solubility of bisacodyl was higher than that in the presence of bisacodyl alone. Furthermore, its solubility is increased as a concentration of dioctyl sodium sulfosuccinate in the solution increases. The results indicate that when preparing a core particle in this formulation, a content of dioctyl sodium sulfosuccinate in the core particle can be increased to improve solubility of bisacodyl and thus to enhance its cathartic effect.

Test Example 5: Effect of combining bisacodyl and DSS
1. Test procedure
   SDmale rats were used in 10 animals per group. Test substances used were bisacodyl alone (2 mg/kg), DSS alone (3.2 mg/kg) and bisacodyl (2 mg/kg)+DSS (3.2 mg/kg).
   1) Intra-ileal administration test
      A rat underwent laparotomy under ether anesthesia and a test substance was injected via a syringe into an ileum at 20 cm to the mouth from the ileocecum at a dose of 1 mL/kg rat weight. After closing the abdomen, the animal recovered from anesthesia and onset of catharsis was observed for 24 hours.
   2) Oral administration test
      A test substance was administered to a rat at a dose of 5 mL/kg weight and onset of catharsis was observed for 24 hours.

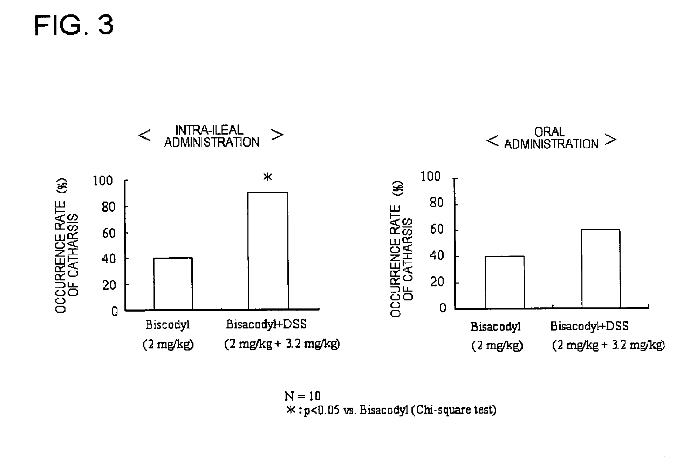

The results are shown in FIG. 3. In both administration routes, cathartic effect was not observed for DSS alone (3.2 mg/kg).

In the intra-ileal administration, combination of bisacodyl with DSS exhibited synergistic enhancement in cathartic effect. In contrast, in the common oral administration, combination with DSS did not exhibited enhancement in cathartic effect of bisacodyl.

These results suggest that a formulation according to the present invention release the drugs, bisacodyl and DSS, in higher concentrations in a region from a lower small intestine to a large intestine and thus their cathartic effect is synergistically enhanced in comparison with effect of each drug alone.

### INDUSTRIAL APPRICABILITY

This invention allows a poorly-soluble drug such as bisacodyl to be effectively delivered in a high concentration to a drug action site and to synergistically improve its effect, and thus can be applied to a pharmaceutical product and the like.

## Claims

1. A solid formulation comprising a core containing bisacodyl and dioctyl sodium sulfosuccinate coated with an enteric coating which allows the drugs to be released in a region from a lower small intestine to a large intestine.

2. The solid formulation as claimed in Claim 1, wherein the core contains light anhydrous silicic acid.

3. The solid formulation as claimed in Claim 1 or 2, wherein the formulation is insoluble in a first fluid for 2 hours and has a disintegration time of more than 60 minutes in a second fluid in accordance with Disintegration Test, Japanese Pharmacopoeia.
